# FASCICULE DE BREVET EUROPEEN

(11) **EP 1 188 070 B1**
(45) Date de publication et mention de la délivrance du brevet: **26.07.2006**
(21) Numéro de dépôt: 00945989.2
(22) Date de dépôt: 22.06.2000
(51) Int. Cl.: G01T 1/161, G01T 1/164

(54) **DISPOSITIF DE DETECTION ET DE LOCALISATION D'UNE SOURCE RADIOACTIVE EMETTRICE DE RAYONNEMENTS GAMMA, UTILISATION DUDIT DISPOSITIF**
VORRICHTUNG ZUM NACHWEIS UND ZUM ORTEN EINER RADIOAKTIVEN, GAMMASTRAHLUNG AUSSENDENDEN QUELLE UND DEREN VERWENDUNG
DEVICE FOR DETECTING AND LOCATING A RADIOACTIVE SOURCE EMITTING GAMMA RAYS, USE OF SAME

(30) Priorité: 22.06.1999 FR 9908115
(43) Date de publication de la demande: 20.03.2002
(73) Titulaire: Maublant, Jean, 63000 Clermont-Ferrand (FR)
(72) Inventeur: Maublant, Jean, 63000 Clermont-Ferrand (FR)
(74) Mandataire: Vuillermoz, Bruno
(86) Numéro de dépôt international: PCT/FR2000/001746
(87) Numéro de publication internationale: WO 2000/079301

(56) Documents cités:
- US-A- 2 942 109
- US-A- 3 794 840

## Description

L'invention se rapporte à un appareil de détection et de localisation d'une source radioactive émettrice de rayonnements gamma. Elle concerne également l'utilisation du dispositif.

L'appareil de détection de l'invention s'applique plus particulièrement dans la suite de la description, mais de façon non limitative, à la détection des ganglions lymphatiques, dont certains en fixant une substance radioactive injectée in situ, sont susceptibles de caractériser l'extension d'une tumeur, notamment dans le cadre du cancer du sein ou du cancer de la peau (mélanome).

Toutefois, d'autres applications du dispositif peuvent être envisagées, telles que la recherche de sources radioactives perdues, le perçage d'un matériau à partir d'indications disponibles sur son autre face et plus largement, la détection et la localisation d'une source radioactive quasi ponctuelle à travers un milieu n'absorbant pas totalement les rayonnements gamma qu'elle émet, et ce, sans que ce soit limitatif.

Les cancers primitifs présentent d'abord un stade local suivi plus ou moins tardivement de localisations secondaires. Le processus d'extension débute avant même que les manifestations cliniques de disséminations ne soient décelables. Les premières structures anatomiques atteintes en dehors de la tumeur primitive sont généralement les ganglions lymphatiques. Aussi, un traitement du cancer consiste à procéder à une ablation des ganglions lymphatiques de la région concernée. Une telle opération vise non seulement à supprimer d'éventuelles localisations secondaires, mais également à diagnostiquer une éventuelle dissémination métastasique permettant ainsi de proposer l'indication d'un traitement médical complémentaire.

Dans le cas particulier du cancer du sein, le traitement chizurgical consiste outre le fait de supprimer la tumeur primitive à retirer également les ganglions lymphatiques situés dans le creux axillaire, opération dénommée "curage lymphatique", puis à procéder à une analyse histologique des ganglions prélevés.

Une telle opération est une source importante de séquelles fonctionnelles pour la patiente. Tout d'abord, l'engorgement lymphatique résultant de la suppression du réseau lymphatique drainant la majeure partie du membre supérieur engendre une pathologie désignée lymphoedème ou "syndrome du gros bras" à l'origine d'une gêne autant fonctionnelle qu'esthétique. De plus, il arrive que lors du curage, les branches des premiers nerfs intercostaux présents dans la région axillaire soient sectionnés conduisant ainsi à l'apparition de troubles sensitifs. On relève également l'apparition de troubles moteurs de l'épaule.

En outre et surtout, il s'avère que les ganglions prélevés lors du curage axillaire ne se révèlent pas toujours envahis à l'analyse histologique. On note notamment que dans le cas des tumeurs de taille inférieure ou égale à 10 mm, seuls 6 % des ganglions prélevés sont envahis. En d'autres termes, 95 % des curages réalisés sont en fait inutiles.

C'est la raison pour laquelle un certain nombre de techniques ont été développées pour tenter de détecter en pré-opératoire et/ou en per-opératoire, puis de retirer, uniquement le ou les ganglions lymphatiques susceptibles d'être atteints lors de l'extension secondaire d'une tumeur primitive.

C'est ainsi qu'on a constaté dans le cadre du traitement du mélanome que le premier relais ganglionnaire drainant le mélanome (dénommé ganglion sentinelle) était susceptible de fixer un colorant, en particulier le bleu patenté.

Il a été démontré que l'absence d'envahissement du ganglion sentinelle correspondait dans près de 100 % des cas à une absence d'envahissement des autres ganglions. Il devenait donc possible en procédant à une analyse extemporanée du ganglion sentinelle repéré puis prélevé, de décider en cours d'intervention de la poursuite ou non du curage ganglionnaire.

Toutefois, selon cette technique, le repérage du ganglion sentinelle étant strictement visuel, il est nécessaire de disséquer les tissus dans la région supposée de la présence du premier relais ganglionnaire. Or, le relais ganglionnaire n'ayant pas de localisation parfaitement définie sur le plan anatomique, cette recherche n'est pas simple et peut s'avérer longue et relativement délabrante.

Pour résoudre ce problème, on a proposé de remplacer le colorant par un produit radioactif. La détection du ganglion sentinelle se fait alors selon deux techniques, respectivement :
- soit par imagerie scintigraphique (lymphoscintigraphie) à l'aide d'une caméra à scintillation ;
- soit à l'aide d'une sonde de détection peropératoire.

Il s'avère que l'imagerie scintigraphique des ganglions lymphatiques, bien que validée dans la visualisation des ganglions en pathologie non cancéreuse, est mal adaptée à la détection spécifique du ganglion sentinelle.

En effet, du fait de sa masse imposante (supérieure à une tonne), l'appareil d'imagerie ne peut être transporté, de sorte que l'examen se déroule nécessairement en pré-opératoire et ailleurs que sur le lieu de ladite opération, compliquant ainsi l'organisation du planning des différents intervenants.

Par ailleurs et surtout, le repérage se fait par l'intermédiaire d'un marquage cutané guidé par une source radioactive mise en correspondance avec la zone hyperfixante sur l'image. De la sorte, cette marque est nécessairement imprécise, puisque :
- d'une part, elle dépend de l'angulation de la tête de la caméra,
- et d'autre part, il est souvent difficile de marquer la peau avec précision dans une région molle et peu accessible ;
- enfin, le chirurgien ne peut pas vérifier en temps réel le bien-fondé du repère cutané.

Parallèlement, l'angle d'incidence de l'incision peut être différent de celui de l'imagerie entraînant une erreur de localisation qui est d'autant plus importante que le ganglion est plus profond.

En d'autres termes, la caméra à scintillation ne permet qu'un repérage préalable grossier de l'emplacement du ganglion en préopératoire.

A l'inverse, la sonde de détection est un matériel de petite taille qui peut donc être manipulé manuellement. Cette sonde détecte les photons gamma émis par une source radioactive et permet le repérage sonore de ladite source placée dans l'angle d'ouverture de son collimateur.

Techniquement, la sonde de détection comprend un collimateur constitué d'un seul trou, un cristal scintillant, un photomultiplicateur à une seule anode et des moyens électroniques associés permettant d'apprécier par une donnée numérique affichée ou un signal sonore l'intensité du signal détecté. Toutefois, cette sonde de détection permet seulement d'apprécier la quantité de radioactivité présente dans le champ de vision de son collimateur, sans être capable de préciser la direction exacte dans laquelle se trouve la source radioactive.

De plus, le repérage du ganglion est très délicat du fait de l'angle d'observation très restreint du système et du grand nombre de degrés de liberté dans le positionnement. Par ailleurs, en raison de la variabilité de l'angle d'incidence, et donc de l'épaisseur des tissus traversés par le rayonnement issu du ganglion, ainsi que de l'instabilité du positionnement, la sonde étant tenue à la main, le repérage ganglionnaire demande beaucoup de temps et s'avère parfois infructueux.

Les documents US-A-2 942 109 et US-A-3 794 840 décrivent des sondes colimatées gamma fournissant uniquement un taux de comptage. Le mouvement de la sonde est soit manuel, soit mécanique (balayage linéaire suivant un déplacement boustrophédon), mais en aucun cas spontané. Il n'existe donc aucun retour d'informations et aucune influence de la position des sources observées sur les mouvements de la tête de détection. A la différence du document US-A-2 942 109, le document US-A-3 794 840 est monté devant un appareil de radiothérapie, mais la conception reste la même.

En d'autres termes, le problème que vise à résoudre l'invention est de proposer un appareil qui permette en pré-opératoire ou surtout en per-opératoire de détecter et de localiser exactement une source radioactive permettant ainsi, dans le cas par exemple de la détection de ganglions lymphatiques, d'éviter tout délabrement inutile en vue ou au cours de l'opération chirurgicale.

Pour résoudre ce problème, l'invention propose un dispositif de détection et de localisation d'une source radioactive émettrice de rayonnements gamma.

Ce dispositif se caractérise en ce qu'il comprend :
- des premiers moyens pour déterminer la direction de la source émettrice des rayonnements gamma par rapport au centre du détecteur ;
- des seconds moyens aptes à pointer la source d'émission radioactive.

En d'autres termes, l'invention consiste en un système de détection permettant en cours d'intervention chirurgicale de repérer et de localiser exactement des organes ou des tissus et notamment des ganglions lymphatiques fixant une source radioactive émettrice de rayonnements gamma.

Contrairement à la technique d'imagerie avec caméra à scintillation, l'objectif n'est pas de constituer une image fidèle du tissu ou organe fixant la source radioactive, mais de repérer dans quelle direction se trouve ladite source par rapport au centre du détecteur, et cela afin de commander la mise en mouvement du détecteur en vue de ramener cette source en face de la partie centrale de la zone de détection.

Pour remplir cet objectif, lesdits premiers moyens comprennent :
◆ des moyens capteurs des rayonnements gamma ;
◆ une pluralité de moyens d'évaluation du flux des rayonnements gamma ;
◆ des moyens d'analyse du flux des rayonnements gamma aptes à déterminer la direction de la source radioactive.

En pratique, les moyens capteurs de rayonnements gamma sont constitués par un collimateur et un cristal scintillant apte à émettre un signal lumineux sous l'effet d'un rayon gamma.

Pour capter les rayons gamma émis par la source et ce, quelle que soit leur direction, le collimateur présente une zone centrale comportant une pluralité de canaux parallèles entre eux et perpendiculaires à la surface dudit collimateur.

Avantageusement, les canaux parallèles sont au nombre de quatre.

Selon une autre forme de réalisation, le collimateur présente en outre une zone périphérique comportant une pluralité de canaux divergents, présentant avec les canaux de la zone centrale une angulation croissante avec leur éloignement par rapport à la zone centrale.

La structure spécifique du collimateur permet donc, de par sa partie périphérique, de détecter l'emplacement d'une source éventuellement lointaine sans se préoccuper de la précision de la localisation et d'une partie centrale présentant une résolution spatiale élevée pour que le calage ultérieur sur la direction du tissu ou organe fixant la source radioactive soit précis.

On peut enfin envisager la mise en oeuvre d'un collimateur asymétrique constitué de canaux divergents tronqués.

Comme déjà dit, le collimateur est accolé sur sa face postérieure à un cristal scintillant apte à émettre un signal lumineux sous l'effet de rayons gamma. Ce cristal est en pratique constitué d'un cristal d'iodure de sodium ou de césium activé au thallium. Il présente une épaisseur suffisante pour arrêter une proportion importante des photons gamma à détecter. En pratique, l'épaisseur du cristal est comprise entre 5 et 10 mm pour la détection de photons d'énergie égale à 140 keV émis par le technétium-99m. Le cristal scintillant présente une forme généralement ronde, dont le diamètre dépend du photomultiplicateur utilisé décrit par la suite.

Comme déjà dit, la précision de la localisation des impacts reçus par la partie périphérique est sans importance. Il suffit de savoir si le détecteur doit être déplacé plutôt dans une direction que dans une autre.

Pour évaluer le flux des rayonnements gamma captés par le collimateur, le dispositif de l'invention comprend une pluralité de moyens d'évaluation constituée par un ou plusieurs photomultiplicateurs à une seule anode aptes à recevoir le signal lumineux émis par les moyens capteurs.

Avantageusement, les photomultiplicateurs à une seule anode sont au nombre de quatre.

Dans une autre forme de réalisation avantageuse du dispositif, les moyens d'évaluation du flux des rayonnements gamma sont constitués par un photomultiplicateur multi-anodes. Le signal issu de chaque anode sera alors considéré équivalent à celui issu de l'anode d'un photomultiplicateur à anode unique.

Pour déterminer exactement la direction de la source radioactive, les premiers moyens comprennent, comme déjà dit, des moyens d'analyse du flux de rayonnement gamma évalué par les moyens d'évaluation. Le but de cette analyse est de déterminer dans quelle direction par rapport au centre du champ de détection se trouve la source radioactive. L'analyse indique que la source est soit décalée dans une direction particulière, soit située dans l'axe du détecteur. L'analyse est effectuée par comparaison des signaux issus des moyens d'évaluation et notamment des photomultiplicateurs. Cette analyse utilise des moyens électroniques pour déterminer parmi les signaux générés par chacun des photomultiplicateurs, ou par groupe de photomultiplicateurs, lequel a la plus grande intensité.

Dans une forme de réalisation avantageuse, suivant le principe de la caméra de Anger, les signaux issus de l'anode de chaque photomultiplicateur à anode unique ou de chaque anode du photomultiplicateur multi-anodes sont combinés pour former quatre signaux codés en X et Y par rapport au centre du photomultiplicateur, notés X-, X+, Y-, Y+, et dont la combinaison fournit des coordonnées X et Y d'un point donné. Ainsi, les intensités détectées sont stockées dans quatre registres, par exemple Nord, Sud, Est, Ouest, en fonction de la positivité de X et de Y.

Lorsqu'il s'agit de traiter l'information uniquement des signaux issus de la zone centrale du cristal, seuls sont stockés les signaux dont la valeur absolue est inférieure à un seuil fixé au préalable.

Dans une forme de réalisation avantageuse, les moyens d'analyse du flux évalué sont aptes à prendre en compte l'intégralité ou une partie seulement des intensités issues des moyens d'évaluation en fonction du degré de quasi uniformisation.

En d'autres termes, le traitement de l'information est effectué tout d'abord à partir des signaux issus de l'ensemble de la surface sensible du cristal, permettant de rapprocher rapidement le centre du détecteur de la source située dans le champ d'exploration, puis ensuite uniquement à partir de la partie centrale du cristal, permettant de centrer plus précisément, mais plus lentement le détecteur sur le point chaud quelles que soient les sources situées dans le reste du champ d'exploration.

Pour permettre de pointer la source d'émission radioactive, lorsque la direction d'émission du rayonnement gamma est détectée et que l'émission d'un signal est intervenue, lesdits seconds moyens de l'appareil de détection de l'invention comprennent :
◆ des moyens mécaniques aptes à imprimer un déplacement au dispositif, de façon à rapprocher son axe central de la source radioactive émettrice de rayonnements gamma ;
◆ des moyens aptes à matérialiser l'axe central du dispositif.

En pratique, les moyens mécaniques aptes à imprimer un déplacement au dispositif répondent à des instructions de déplacement tant que les moyens d'analyse indiquent que l'axe central du détecteur n'est pas en correspondance avec la source.

Par ailleurs, les moyens aptes à matérialiser l'axe central du dispositif se présentent sous forme d'un rayon lumineux.

Selon une forme avantageuse de réalisation, lesdits moyens se présentent sous forme d'au moins deux sources lumineuses cohérentes générant des faisceaux lumineux étroits dont l'intersection correspond à la perpendiculaire au centre de la surface sensible du collimateur (centre du dispositif).

Dans une forme de réalisation particulière, la source lumineuse est positionnée de telle sorte que le bord du faisceau corresponde à une limite de la région explorée par le collimateur.

Comme déjà dit, la dispositif de l'invention peut être utilisé pour la détection d'un tissu ou organe humain ou animal fixant une source radioactive émettrice de rayonnements gamma.

Dans le cas du cancer de la peau ou du cancer du sein, le dispositif sera plus particulièrement utilisé pour la détection des ganglions lymphatiques qui en fixant une source radioactive émettrice de rayonnements gamma témoignent de l'extension secondaire de la tumeur primitive.

Le dispositif pourra également être utilisé pour le repérage peropératoire d'une anomalie telle que par exemple des microcalcifications du sein, qui auront été préalablement marquées par une injection in situ d'une substance radioactive sous contrôle d'imagerie radiographique ou échographique.

Plus généralement, le dispositif de l'invention pourra être utilisé pour la détection et la localisation d'une source radioactive quasi ponctuelle à travers un milieu n'absorbant pas totalement les rayonnements gamma qu'elle émet.

L'invention se rapporte enfin à une méthode de détection d'une source radioactive émettrice de rayonnements gamma fixée par un tissu ou organe humain
ou animal, selon laquelle :
- on injecte une substance radioactive émettrice de rayonnements gamma dans l'organisme ;
- on détermine la direction de la source émettrice des rayonnements gamma ;
caractérisée en ce qu'on matérialise la direction déterminée de manière à pointer la source d'émission radioactive.

L'invention et les avantages qui en découlent ressortiront mieux de l'exemple de réalisation suivant à l'appui des figures annexées.
La figure 1 est une représentation schématique des premiers moyens constitutifs du dispositif de détection de l'invention.
La figure 2 est une coupe du collimateur selon son axe central.
La figure 3 est une représentation des différents impacts détectés par le photomultiplicateur via la surface du collimateur et transformés en signaux électriques.

### Dispositif

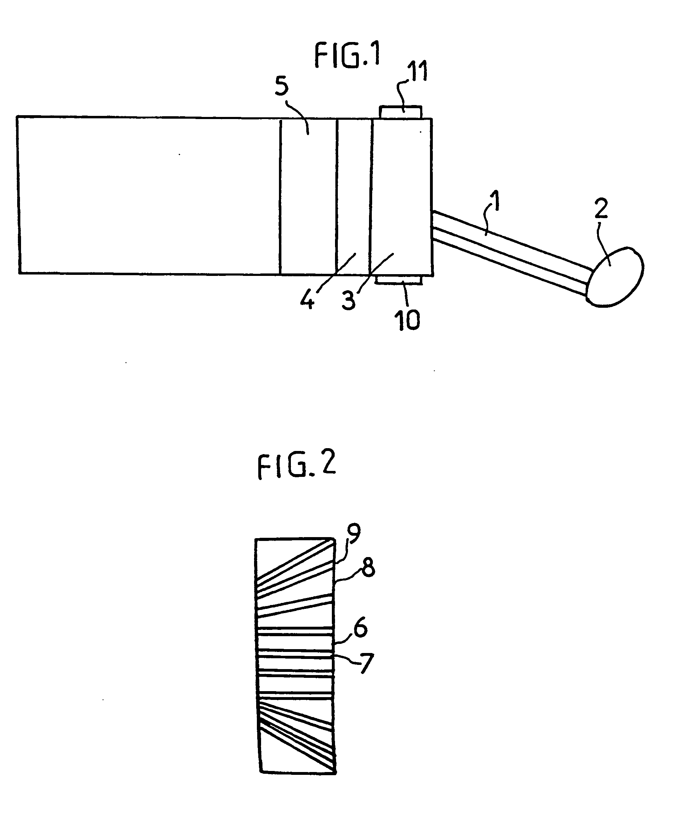

On a représenté de façon schématique sur la figure 1 les premiers moyens du dispositif de détection de l'invention. Ceux-ci sont constitués de moyens capteurs des rayonnements gamma (1) émis par la source (2), d'une pluralité de moyens d'évaluation du flux des rayons gamma et de moyens d'analyse du flux évalué.

Les moyens capteurs sont constitués d'un collimateur (3), lequel présente sur sa face postérieure un cristal scintillant (4).

Par ailleurs, la pluralité de moyens d'évaluation du flux des rayonnements gamma (1) se présentent sous forme d'un photomultiplicateur (5) multi-anodes présentant quatre anodes. Pour repérer dans quelle direction se trouve la source radioactive (2) par rapport au centre du détecteur, le collimateur présente :
- une zone centrale (6) comportant une pluralité de canaux parallèles entre eux (7) et perpendiculaires à la surface dudit collimateur ;
- une zone périphérique (8) comportant une pluralité de canaux divergents (9) présentant avec les canaux (7) de la zone centrale (6) une orientation croissante avec leur éloignement par rapport à ladite zone centrale.

Comme déjà dit, le photomultiplicateur présente quatre anodes délimitant quatre secteurs Nord, Sud, Est, Ouest, par rapport au centre du photomultiplicateur. Ainsi, l'analyse des intensités évaluées par les différents secteurs du photomultiplicateur est réalisée sous forme de quatre points codés en X et Y par rapport au centre du photomultiplicateur, déterminant ainsi les coordonées d'impacts détectées dans les quatre registres Nord, Sud, Est, Ouest.

Le dispositif comprend également des seconds moyens non représentés aptes à pointer la source d'émission radioactive. Ces seconds moyens sont constitués de moyens mécaniques permettant d'imprimer un déplacement au dispositif et des moyens aptes à matérialiser l'axe central du dispositif, notamment sous forme d'un rayon lumineux.

### Mise en oeuvre du dispositif

Dans cet exemple, l'appareil de détection est utilisé pour la détection du ganglion sentinelle marqué au technétium 99 m, notamment dans le cadre d'un cancer du sein.

Pendant l'intervention, l'appareil de détection est placé à quelques centimètres au dessus de la région à l'intérieur de laquelle le ganglion radioactif est vraisemblablement situé.

Dans une première étape, les moyens d'analyse des intensités évaluées par le photomultiplicateur traitent l'intégralité des signaux issus de l'ensemble de la surface sensible du cristal.

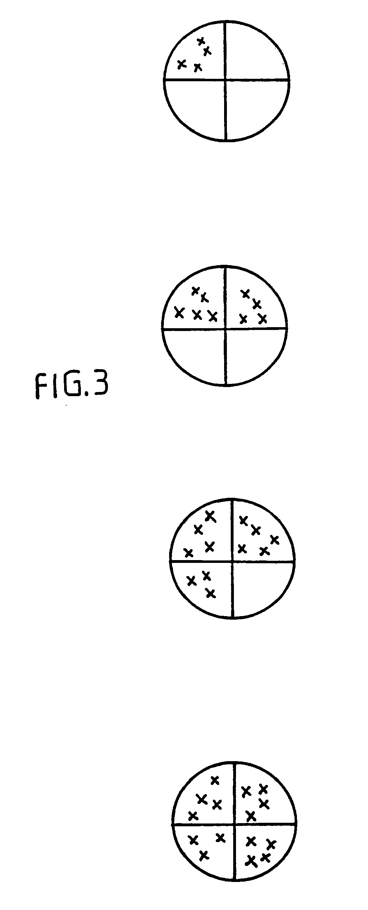

On obtient un certain nombre d'impacts sur les quatre anodes traduits en signaux électriques par le photomultiplicateur. Ces impacts sont répartis dans les quatre registres Nord, Est, Sud, Ouest comme représentés sur la figure 3. Le dispositif est déplacé par les moyens mécaniques dans la direction dans laquelle se trouve le secteur dont l'activité ou flux est supérieur à un seuil minimal de bruit de fond mesuré au préalable. De même, si deux secteurs ont une activité supérieure au bruit de fond, le dispositif se déplace dans la direction intermédiaire entre les deux secteurs jusqu'à obtenir une quasi uniformisation des flux évalués. Si trois ou quatre secteurs ont une activité supérieure, l'appareil traite ensuite les impacts uniquement obtenus dans la zone centrale du cristal.

Afin d'éviter toute remise à zéro des registres à chaque déplacement du dispositif, la remise à zéro peut avoir lieu à intervalles réguliers, notamment toutes les deux secondes. Comme déjà dit, la seconde étape s'enchaîne automatiquement et est restreinte à l'analyse de la partie centrale du cristal, permettant ainsi de centrer plus précisément, mais plus lentement l'appareil sur le point chaud, quelles que soient les sources situées dans le reste du champ d'exploration.

Tant que les valeurs des registres Nord, Est, Sud et Ouest ne sont pas égales, les moyens mécaniques de déplacement sont activés dans la direction du secteur correspondant au registre contenant la valeur maximale. Les quatre registres cardinaux sont remis à zéro à des intervalles de temps plus courts qu'en phase initiale.

Lorsque le tissu ou organe radioactif est au centre du champ, les valeurs contenues dans les registres sont égales entre elles, ce qui immobilise l'appareil et génère un signal spécifique, par exemple un signal sonore ou un clignotement lumineux. Il est à noter toutefois que la notion d'égalité entre la valeur des registres doit tenir compte du fait qu'elle résulte de l'accumulation temporelle d'un nombre de désintégrations radioactives. Ces valeurs sont donc sujettes à des fluctuations statistiques de type poissonien. Il faut donc éviter tout déplacement de l'appareil alors qu'il est en position d'équilibre central, et définir un seuil de différence entre le registre au dessous duquel il est probable que la position d'équilibre est atteinte.

Dans ce but, un cinquième registre noté sigma contient la somme des quatre autres. Une fois l'équilibre atteint, l'axe central du détecteur est matérialisé par un faisceau lumineux du type laser émis par les systèmes (10,11) représentés sur la figure 1. Le faisceau lumineux indique à la surface de la peau ou sur la surface visible des tissus visés la direction de provenance de la source, c'est-à-dire la direction dans laquelle se situe le ganglion radioactif.

Le système présente l'avantage de fonctionner en temps réel, de telle sorte que le faisceau lumineux indique en permanence la direction dans laquelle se trouve le ganglion, même en cas de déplacement éventuel des tissus imprimés par le chirurgien, par exemple lorsqu'il incise des tissus, les palpe ou modifie l'emplacement des écarteurs.

Comme déjà dit, l'appareil de l'invention peut être appliqué à la détection de tous tissus ou organes fixant une source radioactive.

Ce mode de détection sera plus particulièrement avantageux dans la détection des ganglions sentinelles, notamment dans le cadre des cancers du sein ou des mélanomes.

Les avantages de l'invention ressortent bien de la description.

On notera en particulier la capacité de l'appareil à localiser exactement une source radioactive de sorte à limiter au maximum l'intervention chirurgicale.

## Revendications

1. Dispositif de détection et de localisation d'une source radioactive émettrice de rayonnements gamma comprenant :
- des moyens capteurs des rayonnements gamma (1) ;
- une pluralité de moyens d'évaluation aptes à recevoir le signal lumineux émis par les moyens capteurs ;
- des moyens aptes à matérialiser l'axe central du dispositif, notamment sous forme d'un rayon lumineux ;
**caractérisé en ce qu'**il comprend en outre des moyens d'analyse effectuant une comparaison des signaux issus des moyens d'évaluation pour déterminer la direction dans laquelle la source est décalée par rapport à l'axe de détection et des moyens mécaniques aptes à imprimer un déplacement au dispositif, en réponse à des instructions de déplacement tant que les moyens d'analyse indiquent que l'axe central du détecteur n'est pas en correspondance avec la source.

2. Dispositif selon la revendication 1, **caractérisé en ce que** les moyens pour capter les rayons gamma (1) sont constitués par un collimateur (3) et un cristal scintillant apte à émettre un signal lumineux sous l'effet d'un rayon gamma.

3. Dispositif selon la revendication 2, **caractérisé en ce que** le collimateur (3) présente une zone centrale (6) comportant une pluralité de canaux parallèles entre eux (7) et perpendiculaires à la surface dudit collimateur (3).

4. Dispositif selon la revendication 3, **caractérisé en ce que** la zone centrale (6) présente quatre canaux.

5. Dispositif selon l'une des revendications 3 et 4, **caractérisé en ce que** le collimateur (3) présente en outre une zone périphérique (8) comportant une pluralité de canaux divergents (9), présentant avec les canaux de la zone centrale (6) une orientation croissante avec leur éloignement par rapport à la zone centrale (6) .

6. Dispositif selon la revendication 1, **caractérisé en ce que** la pluralité de moyens pour évaluer le flux des rayons gamma est constituée par une pluralité de photomultiplicateurs à anode unique aptes à recevoir le signal lumineux émis par les moyens capteurs (1).

7. Dispositif selon la revendication 6, **caractérisé en ce que** les photomultiplicateurs sont au nombre de quatre.

8. Dispositif selon la revendication 1, **caractérisé en ce que** la pluralité de moyens d'évaluation du flux des rayonnements gamma est constituée par un photomultiplicateur multi-anodes.

9. Dispositif selon la revendication 1, **caractérisé en ce que** les moyens d'analyse du flux de rayonnements gamma sont des moyens électroniques.

10. Dispositif selon la revendication 1, **caractérisé en ce que** les moyens aptes à matérialiser l'axe central du dispositif se présentent sous forme d'au moins deux sources lumineuses cohérentes générant des faisceaux lumineux étroits dont l'intersection correspond à la perpendiculaire au centre de la surface sensible du dispositif.

11. Utilisation du dispositif selon l'une des revendications 1 à 10 pour la détection d'une source radioactive émettrice de rayonnements gamma fixée par un tissu ou organe humain ou animal.

12. Utilisation du dispositif selon la revendication 11 pour la détection d'une source radioactive émettrice de rayonnements gamma fixée par les ganglions lymphatiques.

## Claims

1. An apparatus for detecting and locating a radioactive source emitting gamma rays, comprising:
• gamma-ray (1) sensor means;
• a plurality of measuring means capable of receiving the light signal emitted by the sensor means;
• means capable of defining the central axis of the apparatus, especially in the form of a light ray;
**characterized in that** it furthermore includes analyzing means which compare the signals output by the measuring means in order to determine the direction in which the source is offset with respect to the detection axis, and mechanical means capable of imparting a movement to the apparatus in response to movement instructions as long as the analyzing means indicate that the central axis of the detector is not in correspondence with the source.

2. The apparatus as claimed in claim 1, **characterized in that** the means for sensing the gamma rays (1) consist of a collimator (3) and a scintillating crystal capable of emitting a light signal under the effect of a gamma ray.

3. The apparatus as claimed in claim 2, **characterized in that** the collimator (3) has a central area (6) comprising a plurality of mutually parallel channels (7) which are perpendicular to the surface of said collimator (3).

4. The apparatus as claimed in claim 3, **characterized in that** the central area (6) has four channels.

5. The apparatus as claimed in either of claims 3 and 4, **characterized in that** the collimator (3) furthermore has a peripheral area (8) comprising a plurality of divergent channels (9) making, with the channels of the central area (6), an angle which increases with their distance from the central area (6).

6. The apparatus as claimed in claim 1, **characterized in that** the plurality of means for measuring the gamma-ray flux consists of a plurality of single-anode photomultipliers capable of receiving the light signal emitted by the sensor means.

7. The apparatus as claimed in claim 6, **characterized in that** the number of photomultipliers is four.

8. The apparatus as claimed in claim 1, **characterized in that** the plurality of means for measuring the gamma-ray flux consists of a multi-anode photomultiplier.

9. The apparatus as claimed in claim 1, **characterized in that** the means for analyzing the gamma-ray flux are electronic means.

10. The apparatus as claimed in claim 1, **characterized in that** the means capable of defining the central axis of the apparatus are in the form of at least two coherent light sources generating narrow light beams, the intersection of which corresponds to the perpendicular at the center of the sensitive area of the apparatus.

11. The use of the apparatus as claimed in one of claims 1 to 10 for detecting a radioactive source emitting gamma rays which is fixed by a human or animal tissue or organ.

12. The use of the apparatus as claimed in claim 11 for detecting a radioactive source emitting gamma rays which is fixed by the lymph nodes.

## Patentansprüche

1. Vorrichtung zur Erfassung und Lokalisierung einer Gammastrahlen ausstrahlenden radioaktiven Quelle mit:
- Gammastrahlen-Erfassungsmitteln (1);
- einer Mehrzahl von Auswertemitteln, die dazu geeignet sind, das durch die Erfassungsmittel abgestrahlte Lichtsignal zu empfangen;
- Mitteln, die dazu geeignet sind, die Mittelachse der Vorrichtung zu bilden, insbesondere in Form eines Lichtstrahls;
**dadurch gekennzeichnet, daß** sie ferner Analysemittel umfaßt, welche einen Vergleich der durch die Auswertemittel abgegebenen Signale zur Ermittlung der Richtung durchführen, in der die Quelle in bezug auf die Erfassungsachse verlagert ist, und mechanische Mittel umfaßt, die dazu geeignet sind, eine Verlagerung der Vorrichtung als Reaktion auf Verlagerungsbefehle zu bewirken, solange die Analysemittel anzeigen, daß die Mittelachse des Detektors nicht mit der Quelle übereinstimmt.

2. Vorrichtung nach Anspruch 1, **dadurch gekennzeichnet, daß** die Gammastrahlen-Erfassungsmittel (1) von einem Kollimator (3) und einem Szintillationskristall gebildet werden, der dazu geeignet ist, ein Lichtsignal unter der Einwirkung eines Gammastrahls auszustrahlen.

3. Vorrichtung nach Anspruch 2, **dadurch gekennzeichnet, daß** der Kollimator (3) eine mittlere Zone (6) aufweist, welche eine Mehrzahl zueinander und zur Oberfläche des genannten Kollimators (3) rechtwinkliger Kanäle (7) aufweist.

4. Vorrichtung nach Anspruch 3, **dadurch gekennzeichnet, daß** die mittlere Zone (6) vier Kanäle aufweist.

5. Vorrichtung nach einem der Ansprüche 3 und 4, **dadurch gekennzeichnet, daß** der Kollimator (3) ferner eine Umfangszone (8) aufweist, welche eine Mehrzahl divergierender Kanäle (9) umfaßt, die mit den Kanälen der mittleren Zone (6) eine mit ihrer Entfernung in bezug auf die mittlere Zone (6) zunehmende Anwinklung aufweisen.

6. Vorrichtung nach Anspruch 1, **dadurch gekennzeichnet, daß** die Mehrzahl der Mittel zur Auswertung des Flusses der Gammastrahlen durch eine Mehrzahl von Fotovervielfachern mit Einzelanode gebildet wird, die dazu geeignet sind, das durch die Erfassungsmittel (1) abgestrahlte Lichtsignal zu empfangen.

7. Vorrichtung nach Anspruch 6, **dadurch gekennzeichnet, daß** vier Fotovervielfacher vorhanden sind.

8. Vorrichtung nach Anspruch 1, **dadurch gekennzeichnet, daß** die Mehrzahl der Auswertemittel des Flusses der Gammastrahlung von einem Fotovervielfacher mit Mehrfachanode gebildet wird.

9. Vorrichtung nach Anspruch 1, **dadurch gekennzeichnet, daß** die Mittel zur Analyse des Flusses der Gammastrahlen elektronische Mittel sind.

10. Vorrichtung nach Anspruch 1, **dadurch gekennzeichnet, daß** die Mittel, die zur Bildung der Mittelachse der Vorrichtung geeignet sind, die Form mindestens zweier kohärenter Lichtquellen aufweisen, welche schmale Lichtstrahlenbündel erzeugen, deren Kreuzungspunkt der Senkrechten auf die Mitte der empfindlichen Oberfläche der Vorrichtung entspricht.

11. Verwendung einer Vorrichtung gemäß einem der Ansprüche 1 bis 10 zur Erfassung einer Gammastrahlen ausstrahlenden radioaktiven Quelle, welche durch ein menschliches oder tierisches Gewebe oder Organ festgelegt ist.

12. Verwendung einer Vorrichtung gemäß Anspruch 11 zur Erfassung einer Gammastrahlen ausstrahlenden radioaktiven Quelle, welche durch die Lymphknoten festgehalten ist.
